Europäisches Patentamt

European Patent Office

Office européen des brevets

(11) Publication number: **0 540 754 A1**

# EUROPEAN PATENT APPLICATION
## published in accordance with Art. 158(3) EPC

(21) Application number: 92910344.8

(22) Date of filing: 22.05.92

(86) International application number: **PCT/JP92/00660**

(87) International publication number: **WO 92/20318 (26.11.92 92/29)**

(51) Int. Cl.⁵: **A61K 6/10**

(30) Priority: **23.05.91 JP 149873/91**

(43) Date of publication of application: **12.05.93 Bulletin 93/19**

(84) Designated Contracting States: **DE FR GB**

(71) Applicant: **SANKIN KOGYO KABUSHIKI KAISHA**
1382−11, Shimoishigami
Ohtawara−shi, Tochigi 329−26(JP)

(72) Inventor: **YUDA, Sadayuki**
C18−201, 1, Tsukumodai 2−chome
Suita−shi Osaka 565(JP)
Inventor: **KUROKI, Katsujiro**
C−308, 7, Kotesashi−cho 1−chome
Tokorozawa−shi Saitama 359(JP)

(74) Representative: **Vossius & Partner**
Siebertstrasse 4 P.O. Box 86 07 67
W−8000 München 86 (DE)

(54) **DENTAL IMPRESSION MATERIAL COMPRISING ALGINATE.**

(57) A dental impression material comprising an alginate, a gelling agent, a filler and, in addition, liquid paraffin, hydroxypropylocellulose (HPC) and a surfactant. The HPC may be replaced by polyvinyl alcohol (PVA). The surface of the alginate impression material composition can be uniformly coated by uniformly dissolving or suspending the HPC or PVA in a solution of the liquid paraffin and surfactant.

EP 0 540 754 A1

Technical Field:

The present invention relates to a dental alginate impression material which is less dusty, has good storage stability, and provides an impression having a small surface roughness. More particularly, the present invention relates to an improved dental alginate impression material which retains its characteristic properties (such as gel time and compressive strength) even after storage for a long time.

Background Art:

The use of dental alginate impression material generally involves the kneading of certain powdered compositions of the impression material with water, hut it has a disadvantage that when the powdered composition is kneaded with water so as to be applied to a patient, it flies about, offending the technician.

Japanese Patent Publication No.Hei 2 − 54325 discloses a technical idea of making it less dusty by coating with a particular coating material. This technical idea, however, does not consider storage stability and impression's surface roughness at all; therefore, it suffers a disadvantage that the coated impression material absorbs moisture upon contact with the air during its preparation or use, which affects the gel time and the elasticity. And it is also pointed out thatdimensional accuracy of the impression taken is insufficient.

Japanese Patent Publication No.Hei 1 − 42245 discloses a technical idea of improving the impression's surface roughness by comprising poly(vinylpyrrolidone) (hereinafter referred to as PVP), which is a water − soluble polymer. The technique consists of mixing PVP into an impression (composed of alginic acid salt, gelling agent and filler) by dry process, and adding dropwise to the mixture one or more kind of hydrophobic hydrocarbons or silicone oils containing no hydrophilic groups. The resulting impression material, however, does not necessarily meet requirements not only for smooth coating with PVP, but also for storage stability during its preparation and use. Thus there has been a long seek for the development of new technologies.

The present invention was completed in view of the foregoing Accordingly it is an object of the present invention to provide a dental alginate impression material which is less dusty, provides an impression having a small surface roughness and has good storage stability.

Disclosure of the Invention:

The gist of the present invention resides in a dental alginate impression material of the type containing an alginic acid salt, a gelling agent, and a filler, which comprises liquid paraffin, hydroxypropyl cellulose (hereinafter referred to as HPC), and a surface active agent. According to the present invention, HPC may be replaced by poly(vinyl alcohol) (hereinafter referred to as PVA).

Best Mode for Carrying Out the Invention:

As the result of our researches, we found that a dental alginate impression material becomes less dusty, improves in storage stability, and provides an impression having a small surface roughness if the surface of composition of alginate impresssion material is coated smoothly with a solution of HPC or PVA by dissolving or suspending HPC or PVA homogenously into a solution including liquid paraffin and a surface active agent. The present invention was completed on the basis of this finding.

According to the present invention, the dental alginate impression material should contain HPC or PVA in an amount of 0.1 − 8 wt%, preferably 0.3 − 5 wt%, so that it exhibits a good storage stability.

According to the present invention, the ratio of liquid paraffin to surface active agent is preferably from 9:1 to 6:4 (by weight) to ensure homogenous dissolution or suspension of HPC or PVA, and the ratio of a mixture of liquid paraffin and surface active agent to HPC or PVA is preferably from 9:1 to 7:3 (by weight).

Examples of the surface active agent include lauryl sulfate ester, polyoxyethylene oleyl ether, sorbitan fatty acid ester, and alkyldiethanolamine.

The kind of alginic acid salt, gelling agent, and filler is not specifically limited. All which have conventionally been used for alginate impression materials can be used in the present invention.

Examples of the alginic acid salt include sodium alginate, potassium alginate, ammonium alginate, and triethanolamine alginate.

The gelling agent that can be used in the present invention is one which consists of a reacting agent and an adjusting agent, the former being a divalent (or higher) metal salt such as calcium sulfate, the latter being a phosphate, silicate, or carbonate of sodium, calcium, or the like. Examples of the filler include diatomaceous earth, anhydrous silicate, talc, calcium carbonate, pearlite, silica, aluminum hydroxide or the like.

The amounts of the alginic acid salt, gelling agent, and filler are not specifically limited in the present invention. They may be properly selected as in the conventional practice. For example, the alginic acid salt may account for 10 – 30 wt% and the gelling agent 10 – 35 wt%, with the rest being the filler.

EXAMPLES

The invention will be described in more detail with reference to the following examples.

Several kinds of dental alginate impression materials were prepared according to the recipe shown in Table 1. The preparation consists of mixing liquid paraffin with a surface active agent, dissolving or suspending HPC or PVA in the mixture, and adding successively to the mixture a filler (diatomaceous earth), so that a smooth coating being formed ,further adding a gelling agent and an alginic acid salt to the mixture, finally obtaining the alginate impression materials.

Table 1 (parts by weight)

| | | Example No. | | | | | Comparative Example No. | | | | |
|---|---|---|---|---|---|---|---|---|---|---|---|
| | | 1 | 2 | 3 | 4 | 5 | 1 | 2 | 3 | 4 | 5 |
| Alginate | Sodium alginate | 15 | – | 7.5 | 7.5 | 7.5 | 15 | 15 | 15 | 15 | 15 |
| | Potassium alginate | – | 15 | 7.5 | 7.5 | 7.5 | – | – | – | – | – |
| Gelling agent | Calcium sulfate | 15 | 15 | 15 | 15 | 15 | 15 | 15 | 15 | 15 | 15 |
| | Sodium pyrophosphate | 1.5 | 1.5 | 1.5 | 1.5 | 1.5 | 1.5 | 1.5 | 1.5 | 1.5 | 1.5 |
| | Titanium potassium fluoride | 1 | 1 | 1 | 1 | 1 | 1 | 1 | 1 | 1 | 1 |
| Filler | Aluminum hydroxide | 3 | 3 | 3 | 3 | 3 | 3 | 3 | 3 | 3 | 3 |
| | Diatomaceous earth | 64.5 | 64.5 | 64.5 | 64.5 | 64.5 | 64.5 | 64.5 | 64.5 | 64.5 | 64.5 |
| Water–soluble polymer | HPC *1 | 1 | 1 | – | 1 | 0.5 | – | – | – | – | 1 |
| | PVA *2 | – | – | 1 | – | – | – | – | – | – | – |
| | PVP | – | – | – | – | – | – | 1 | 1 | – | – |
| | HEC *3 | – | – | – | – | – | – | – | – | 1 | – |
| Surface active agent | Lauryl sulfate ester | – | – | – | – | 1 | – | – | – | – | – |
| | Polyoxyethylene oleyl ether | 1 | 1 | 1 | – | 1 | – | – | 1 | 1 | – |
| Liquid paraffin | | 8 | 8 | 8 | 8 | 8 | – | 8 | 8 | 8 | – |

*1 HPC made by Nippon Soda Co., Ltd.
     HPC-SSL, 2% aq. solution; viscosity 2.0–2.9 cps (for 1 part by weight)
     HPC-L, 2% aq. solution; viscosity 6–10 cps (for 0.5 part by weight)

*2 PVA made by Kuraray Co., Ltd.
     PVA-205, 4% aq. solution; viscosity 4–6 cps

*3 HEC: hydroxyethyl cellulose

The above – mentioned samples of dental alginate impression material were tested in the following manner for gel time, compressive strength, surface roughness, storage stability, and tendency to fly about at the time of kneading. The results are shown in Table 2. Incidentally, 21 g of the powdered composition of impression material was kneaded with 48 ml of water.

Evaluation method

- Gel time and compressive strength were measured in accordance with JIS (Japanese Industrial Standards) T – 6505 (for dental alginate impression material).
- Surface roughness was evaluated by the method which consists of taking an impression of a smooth acrylic plate (20 x 30 x 5 mm) with the sample of impression material, curing the impression material, immediately after removing the acrylic plate, pouring gypsum, then allowing gypsum to set at room temperature for 1 hour, removing the cured gypsum, and measuring the surface roughness of the impressed surface using a surface shape meter ("Surfcom 50A" manufactured by Tokyo Seimitsu Co., Ltd.).
- Storage stability was evaluated by measuring the gel time and compressive strength of the sample which had been allowed to stand at 25°C and 90 %RH (relative humidity) for 120 hours.

Table 2

| | | Gel time | Compressive strength (MPa) | Storage stability | | Surface roughness (μm) | Flying about at the time of kneading |
|---|---|---|---|---|---|---|---|
| | | | | Gel time | Compressive strength (MPa) | | |
| Example No. | 1 | 2'10" | 0.72 | 2'10" | 0.70 | 7 | none |
| | 2 | 1'55" | 0.73 | 2'00" | 0.70 | 8 | none |
| | 3 | 2'00" | 0.71 | 2'00" | 0.71 | 6 | none |
| | 4 | 2'00" | 0.72 | 2'00" | 0.70 | 6 | none |
| | 5 | 2'00" | 0.71 | 2'05" | 0.69 | 7 | none |
| Comparative Example No. | 1 | 2'10" | 0.72 | 5'10" | 0.50 | 7 | yes |
| | 2 | 2'10" | 0.71 | 3'45" | 0.61 | 7 | none- |
| | 3 | 2'10" | 0.71 | 3'15" | 0.63 | 6 | none |
| | 4 | 2'10" | 0.72 | 3'00" | 0.63 | 7 | none |
| | 5 | 2'10" | 0.71 | 4'30" | 0.53 | 18 | none |

It is noted from Table 2 that the samples in Examples 1 to 5, which conform to the requirements of the present invention, do not fly about at the time of kneading, provide impressions having a small surface roughness, and have a good storage stability. By contrast, the samples in Comparative Examples 1 to 5, which do not meet one or more requirements of the present invention, is poor in storage stability (or the extended gel time and the decreased compressive strength). The sample in Comparative Example 1, which does not contain liquid paraffin, water – soluble polymer, and surface active agent, is poor in storage stability and flies about at the time of kneading. The sample in Comparative Example 2, which contains PVP as a water – soluble polymer and contains no surface active agent, is poor in storage stability. The samples in Comparative Examples 3 and 4, which contain a surface active agent but contain PVP or HEC as a water – soluble polymer, are poor in storage stability. The sample in Comparative Example 5, which contains neither liquid paraffin nor surface active agent, is poor in storage stability and provides an impression having a large surface roughness.

Exploitation in Industry:

Owing to the new composition as mentioned above, the dental alginate impression material of the present invention shows little tendency to fly about at the time of kneading, exhibits a good storage stability during its preparation and use, and provides an impression having a small surface roughness.

## Claims

1. A dental alginate impression material of the type containing an alginic acid salt, a gelling agent, and a filler, characterized by comprising liquid paraffin, hydroxypropyl cellulose, and a surface active agent.

2.  A dental alginate impression material of the type containing an alginic acid salt, a gelling agent, and a filler, characterized by comprising liquid paraffin, poly(vinyl alcohol), and a surface active agent.

# INTERNATIONAL SEARCH REPORT

International Application No PCT/JP92/00660

## I. CLASSIFICATION OF SUBJECT MATTER (If several classification symbols apply, indicate all) [6]

According to International Patent Classification (IPC) or to both National Classification and IPC

Int. Cl$^5$   A61K6/10

## II. FIELDS SEARCHED

| Minimum Documentation Searched [7] | |
|---|---|
| Classification System | Classification Symbols |
| IPC | A61K6/10 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X | JP, A, 61-85306 (Tokuyama Soda Co., Ltd.), April 30, 1986 (30. 04. 86), (Family: none) | 1, 2 |
| Y | JP, A, 60-105607 (Jishi Shika Kogyo K.K.), June 11, 1985 (11. 06. 85), & DE, A, 3439885 & US, A, 4543372 | 1, 2 |
| Y | JP, A, 57-501426 (Dentsply International, Inc.), August 12, 1982 (12. 08. 82), & WO, A1, 82/00650 & US, A, 4394172 | 1, 2 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search | Date of Mailing of this International Search Report |
|---|---|
| July 13, 1992 (13. 07. 92) | August 4, 1992 (04. 08. 92) |
| International Searching Authority | Signature of Authorized Officer |
| Japanese Patent Office | |

Form PCT/ISA/210 (second sheet) (January 1985)